Europäisches Patentamt

European Patent Office

Office européen des brevets

(19)

(11) Numéro de publication : **0 048 531**
**B1**

(12) **FASCICULE DE BREVET EUROPÉEN**

(45) Date de publication du fascicule du brevet :
04.07.84

(21) Numéro de dépôt : **81201059.3**

(22) Date de dépôt : **23.09.81**

(51) Int. Cl.³ : **G 01 L 11/00, A 61 B 5/02**

(54) **Dispositif de mesure de pressions en des endroits peu accessibles.**

(30) Priorité : **24.09.80 CH 7134/80**

(43) Date de publication de la demande :
**31.03.82 Bulletin 82/13**

(45) Mention de la délivrance du brevet :
**04.07.84 Bulletin 84/27**

(84) Etats contractants désignés :
**AT BE DE FR GB IT LU NL SE**

(56) Documents cités :
**DE-A- 1 648 433**
**DE-A- 2 811 859**
**DE-A- 2 816 186**
**DE-B- 1 773 296**
**DE-B- 2 027 071**
**US-A- 3 099 262**

(73) Titulaire : **METABO S.A. Construction d'appareils
scientifiques et médicaux
CH-1066 Epalinges (CH)**

(72) Inventeur : **Favre, Robert
5, chemin du Levant
CH-1005 Lausanne (CH)**

(74) Mandataire : **Meylan, Robert Maurice
c/o Bugnion SA Conseils en Propriété Industrielle 10,
Route de Florissant Case Postale 375
CH-1211 Genève 12 - Champel (CH)**

## Description

La mesure d'une pression est particulièrement simple lorsqu'il s'agit d'un milieu fluide, pouvant pénétrer directement dans le circuit manométrique. Les pressions physiologiques sont, de ce fait, souvent mesurées par l'intermédiaire d'un fluide.

Les gaz présentent l'avantage de ne pas accuser d'effet de pesanteur mais les procédés connus (brevet US-A-3 099 262) ne permettent pas, en raison d'un diamètre trop élevé, d'opérer par cathétérisme, notamment : dans les voies urinaires, l'œsophage, ou le cœur.

La présente invention a précisément pour objet un dispositif de mesure de pressions physiologiques en des endroits peu accessibles, comprenant une sonde, notamment de manocathétérisme, pour transmettre une pression à mesurer mal accessible cette sonde comprenant au moins un circuit pneumatique à flux d'air ou autre gaz, ledit circuit comprenant, un tube filiforme d'entrée, un capteur de transposition de ladite pression à mesurer, et un collecteur d'évacuation, ledit capteur étant pourvu d'au moins une valve comprenant, une membrane élastique, un pavillon, et un drain, ladite valve déterminant une ligne d'obturation sur le sommet dudit pavillon, ladite ligne d'obturation engendrant un gradient de pression dudit flux, tel que la pression dans ledit pavillon soit maintenue adaptée à ladite pression à mesurer, lesdits moyens de mise en œuvre comprenant un propulseur qui assure la circulation dudit flux, un organe d'ajustage du débit dudit flux, et un manomètre, c'est-à-dire du type écrit dans le brevet US-A-3 099 262.

Le dispositif selon l'invention est caractérisé par le fait que, tout en alimentant ledit capteur par ledit flux, ledit tube filiforme joue également le rôle de transmetteur de ladite pression à mesurer jusqu'en amont de ladite sonde, où elle sera mesurée par ledit manomètre, de manière à éviter une tubulure spécifique de mesure et réduire d'autant le diamètre de ladite sonde.

Les dessins annexés représentent, à titre d'exemple, quelques formes d'exécution de l'invention.

La figure 1 schématise le principe général d'un dispositif selon l'invention, et les moyens de mise en œuvre, la membrane dudit capteur étant ici à surface active plane.

La figure 2 donne une coupe longitudinale d'un dit capteur pourvu d'une membrane à surface active cylindrique.

La figure 3 donne une coupe transversale du capteur selon figure 2.

La figure 4 donne une coupe longitudinale du corps d'une variante du capteur selon figure 2.

La figure 5 donne la coupe transversale, au niveau de l'axe A-A, du corps selon figure 4.

La figure 6 représente une pièce figurant en deux exemplaires sur le corps selon figure 5.

La figure 7 est une vue en bout de la pièce selon figure 6.

La figure 8 donne une vue d'ensemble de la variante de capteur obtenue par assemblage des composants selon figures 4 à 7.

La figure 9 donne la coupe transversale, au niveau de l'axe B-B, du capteur selon figure 8.

La figure 10 montre l'assemblage des tubes plastiques adaptés au capteur selon figures 2 ou 8.

La figure 11 schématise l'adaptation d'un dispositif selon l'invention à une sonde pour la mesure de la pression des varices œsophagiennes, à travers un endoscope.

La figure 12 schématise l'adaptation d'un dispositif selon l'invention à la mesure de la pression intracrânienne des prématurés, par l'intermédiaire de la fontanelle.

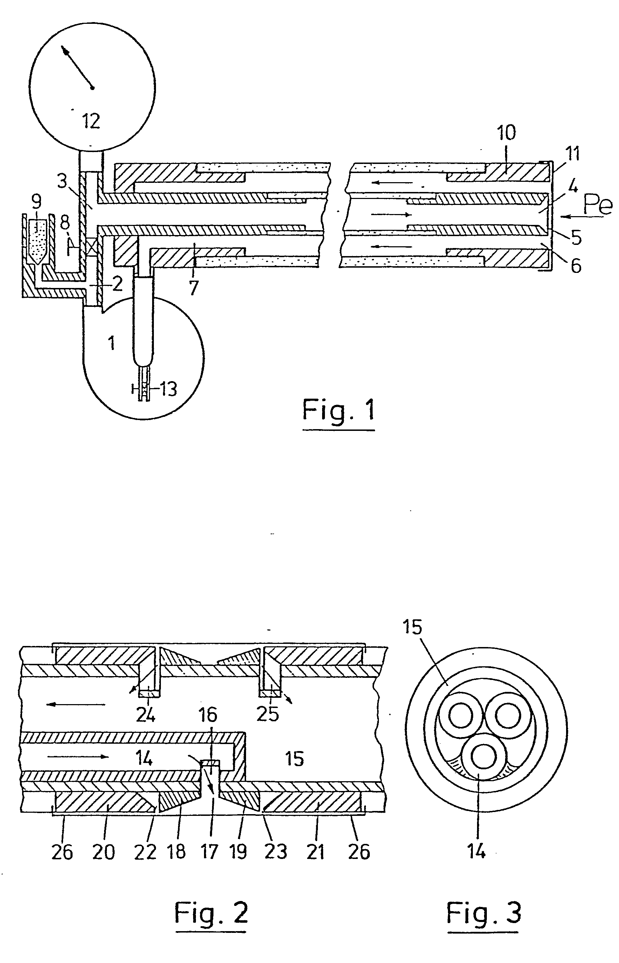

En se référant à la figure 1, le principe de fonctionnement d'un dispositif selon l'invention, se comprend comme suit :

Un flux d'air — propulsé par un mini-compresseur spécifique 1, et dont le débit est ajustable par le robinet 8 — circule dans le circuit pneumatique d'une sonde comprenant, le tube filiforme d'entrée 3-4, le capteur 10, et le collecteur d'évacuation 6-7, avant de retourner, éventuellement, au propulseur 1.

Le capteur 10 est pourvu d'une valve chargée d'équilibrer la pression extérieure Pe à mesurer, par la pression intérieure dudit flux, ladite valve comportant, un pavillon 4, un drain 6, et une membrane élastique 11, agencée de manière à intercepter ledit flux au niveau d'une ligne d'obturation 5 (ici un cercle) aussi longtemps que la pression dudit flux dans le pavillon 4, est inférieure à la pression extérieure Pe.

En appelant, de manière générale, débit « d'alimentation » celui déterminé par le robinet 8, et débit » d'échappement » celui qui passe sous la ligne d'obturation 5, ce dernier sera inférieur au premier lorsque la pression Pe est en augmentation, mais il lui sera supérieur lorsque Pe est en diminution... jusqu'à ce que la pression Pe soit équilibrée par la pression interne.

Désignons, de manière générale, par Px la pression au point » x » dudit circuit pneumatique.

Une solution connue (brevet US-A-3 099 262) consiste à mesurer, par un manomètre extérieur, la pression P4, telle qu'elle est transposée au point 4, par le jeu de la membrane 11... ce qui implique une jonction supplémentaire dans le pavillon 4, et un tube spécifique de mesure, d'où un diamètre total de la sonde incompatible avec les applications physiologiques envisagées, par mano-cathétérisme.

Dans le dispositif selon la présente invention, le tube filiforme 3-4, tout en alimentant la valve du capteur 10 avec ledit flux, joue également le rôle de « transmetteur » de la pression, depuis le cercle d'obturation 5 jusqu'au point 3 situé en amont de ladite sonde, où sa valeur est mesurée par le manomètre 12, après correction des « distorsions » de transfert.

2

Il faut signaler en effet un gradient de pression positif, dans le sens Pe-P5-P4-P3, à raison d'une composante indépendante de Pe, et une autre proportionnelle à Pe.

La composante indépendante de Pe, sous réserve d'un débit constant qui implique Pe beaucoup plus grand que Pe, procède de la perte de charge P3-P4... et peut être résorbée par une simple « mise à zéro » du manomètre 12.

La composante proportionnelle à Pe procède, en revanche, de ce que, la pression P6 étant nécessairement plus faible que Pe, la pression P4 doit être plus grande que Pe pour compenser l'équilibre... d'autant qu'il s'y ajoute, au niveau du cercle d'obturation 5, un effet Venturi et une perte de charge spécifique, également proportionnels à Pe.

Ajoutons que l'effet de la pression P6 est d'autant plus faible que la largeur du drain 6 est plus petite.

La composante proportionnelle à Pe peut être compensée, soit en diminuant la « sensibilité » du manomètre — par exemple au moyen d'un atténuateur sur le circuit d'un transducteur électronique, solution recommandée pour cette application — soit par l'adaptation de P2, pour que la réduction du débit et de la perte de charge P3-P4, consécutives à une hausse de Pe, compense l'effet en cause sur le domaine utile.

Dans un tout autre ordre d'idée, il est évident que la pression P6 doit être toujours inférieure à Pe, ce qui implique souvent qu'elle soit négative par rapport à la pression atmosphérique, ne fût-ce que pour la » mise à zéro » objective du manomètre 12.

A cet effet, la pression P2 est limitée par une soupape 9 alors que la pression sortie-entrée P2-P7 du propulseur 1, est stabilisée par un système connu, non représenté... de telle manière que P7 puisse être réglée à une valeur négative quelconque.

Pour fixer les idées, dans une sonde selon l'invention destinée à mesurer la pression des varices œsophagiennes, à travers un endoscope, la perte de charge dans le collecteur d'évacuation est telle que la pression P7 doit être inférieure à la pression atmosphérique d'environ 150 cm d'eau.

Cette valeur numérique sera obtenue, par exemple, en réglant la soupape 9 à 350 cm d'eau, et la pression P2-P7 à 500 cm d'eau.

S'agissant du « temps de réponse » d'une sonde selon l'invention, on remarquera qu'un saut positif de la pression Pe, grand par rapport à la perte de charge P3-P5, entraîne le blocage du flux au niveau du cercle d'obturation 5... jusqu'à ce que la pression P5 atteigne environ Pe, sous l'effet du débit D, et compte tenu du » volume mort » Vm compris entre le robinet 8 et la ligne d'obturation 5.

En appelant Pr la pression absolue, à mi-hauteur d'un saut sPe de la pression Pe, le temps de réponse Tr du dispositif aura en première approximation la forme :

$$Tr = Vm \cdot sPe/Pr \cdot D$$

Expression qui néglige, tant la dilatation éventuelle du volume mort Vm sous l'effet de la pression, que la progressivité d'une réouverture dudit débit « d'échappement » sous la ligne d'obturation.

Le temps de réponse à un saut de pression sPe négatif, dépendra de l'augmentation consécutive dudit débit « d'échappement », donc notamment de la compliance de la membrane élastique 11, mais il est facile d'obtenir une réponse négative au moins aussi rapide que la réponse positive.

Il reste que le temps de réponse, à un saut de pression, grand par rapport à P3-P5, est approximativement proportionnel à sPe, et au « volume mort » Vm, compris entre le robinet 8 et le cercle d'obturation 5 — donc notamment à la longueur du tube filiforme 3-4 tandis qu'il est inversement proportionnel au débit dudit flux, partant, à la perte de charge P3-P5.

Il y a donc intérêt à tenir le volume mort Vm aussi faible que possible, également en ce qui concerne la liaison avec le manomètre, laquelle sera notamment fait par l'intermédiaire d'un transducteur électronique dont on citera les types 142 PC 05 Q de Honeywell et LX 1601 DF de National.

L'écoulement étant laminaire, le temps de réponse relatif à une perte de charge P3-P5 donnée, est donc sensiblement proportionnel au carré de la longueur du tube filiforme 3-4.

Un tube de nylon de 0,75 mm de diamètre intérieur par exemple, donne, sous perte de charge de 10 cm d'eau par mètre, un débit d'air de 0,09 ml/s, et présente un volume mort de 0,44 ml/m.

Une sonde équipée de la sorte, pour déterminer la motilité œsophagienne, d'une longueur de 1 mètre toutes liaisons annexes comprises, donne, pour une perte de charge de 10 cm d'eau, un temps de réponse d'environ 0,1 seconde.

On peut évidemment réduire ce temps de réponse en augmentant le débit, donc la perte de charge, mais il est encore possible de relever les performances générales en remplaçant le flux d'air par un gaz de viscosité plus faible, tel qu'un hydrocarbure volatile, ou l'ammoniac.

On placera, pour cela, une ampoule contenant le gaz choisi, à l'état liquide (propane) ou dissout (ammoniaque), à l'entrée 13 du propulseur 1, en commençant par un « rinçage » du circuit pneumatique.

Une telle ampoule, mais de forme et structure appropriée, peut encore remplir le rôle de propulseur, par un chauffage asservi, tandis que la pression négative d'aspiration sera obtenu par refroidissement de l'hydrocarbure, ou dissolution de l'ammoniac dans de l'eau plus froide.

Le figure 2 relative à un capteur destiné plus particulièrement à mesurer une pression s'exerçant radialement, par exemple telle qu'elle se présente dans l'urètre.

Ledit flux est introduit dans ledit capteur par un microtube 14 soudé à un corps tubulaire 15, puis il traverse une ouverture latérale 16, se propage dans une gorge circulaire 17 concentrique au corps 15, atteint le pavillon formé des bagues 18 et 19, parvient au drain constitué par les gorges circulaires 22 et 23 également concentriques au corps 15 et reliées par des lumières latérales 24 et 25 au collecteur, lequel commence à l'intérieur du corps 15.

Lesdites bagues sont couvertes par la membrane élastique 26, en forme de gaine cylindrique — par exemple en latex — placée sans jeu ni contrainte... qui détermine une ligne d'obturation répartie en deux cercles périphériques situés sur les bords des bagues 18 et 19, à la limite des gorges 22 et 23.

Ces cercles d'obturation délimitent la bande annulaire sur laquelle s'opère la mesure, à savoir une distance qui peut varier de 0,5 à 2 mm, selon la définition requise.

La compliance utile de la membrane 26 s'exerçant dans le sens d'une dilatation cylindrique, est relativement faible, mais il est possible de l'améliorer en donnant au manteau extérieur du capteur une forme prismatique donc à section polygonale, singulièrement hexagonale, les mouvements de la membrane étant facilités en regard des faces planes qui sont ménagées de la sorte.

La figure 3 montre l'aspect d'un brasage du microtube 14 dans le corps 15 d'un capteur selon figure 2, et illustre la possibilité d'y loger d'autres tubes filiformes en transit, destinés à alimenter autant de capteurs indépendants situés en aval relativement au propulseur 1 d'une sonde dite « multiple ».

Signalons que le diamètre total d'une sonde à trois voies selon figures 2 et 3 — comme celui de chacun de ses capteurs — ne doit, dans certaines applications médicales, pas excéder 3,3 mm, voir moins.

Il arrive que la composante radiale de la pression exercée sur la bande annulaire active d'un tel capteur à membrane élastique cylindrique, ne soit pas isotrope... et une solution selon la figure 2 ne retiendrait alors que sa valeur minimum, comme il résulte de son principe.

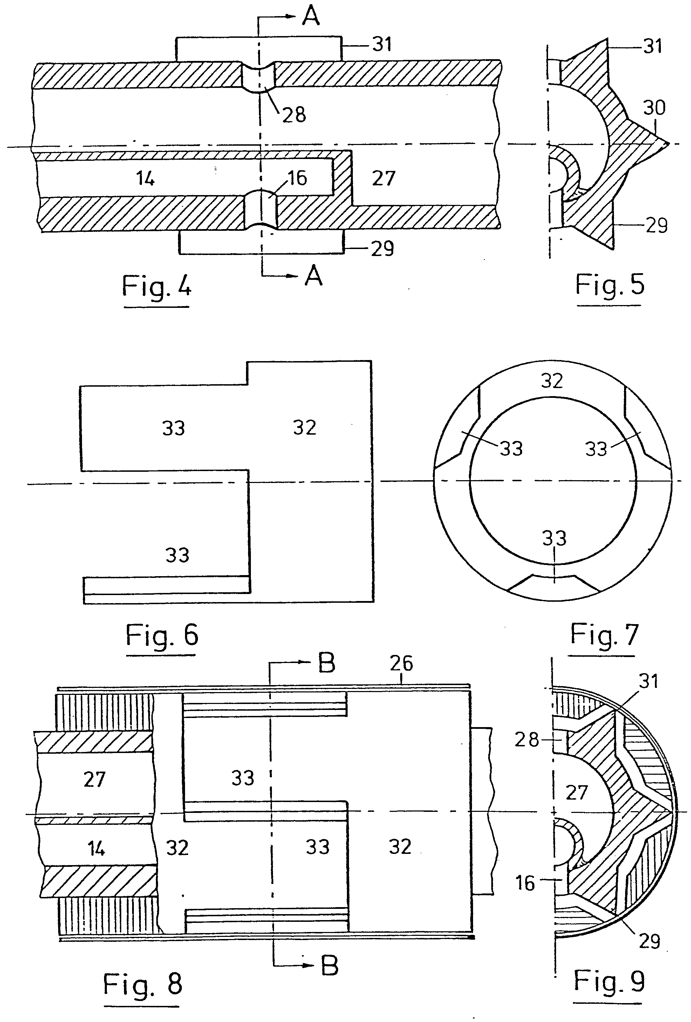

Les figures 4 à 9 sont relatives à une variante qui, en revanche, retiendra la valeur quasi-maximum de cette composante radiale, non isotrope.

Les figures 4 et 5 sont des coupes longitudinale, et transversale selon l'axe A-A, représentant le corps tubulaire 27, homologue du corps 15 de la figure 2, et le microtube d'entrée 14 semblable dans les deux cas.

Le corps 27 porte, en revanche, des dents largement espacées 29 à 31, entre lesquelles viennent s'intercaler les semelles 33 de deux pièces selon figure 6 (vue en long) et 7 (vue en bout) ajustées en tête-bêche, comme le montrent les figures 8 (vue en long), et 9 (vue transversale selon l'axe B-B), tandis que la membrane 26 recouvre le tout comme dans le cas de la figure 2.

Ledit flux, introduit par le microtube 14, traverse l'ouverture latérale 16 (fig. 9) et suit dès lors le profil transversal extérieur du corps 27, notamment des dents 29 à 31, par un espace ménagé sous les semelles 33 (fig. 7) limité latéralement par le bord intérieur de la partie 32, pour atteindre le collecteur 27 par la lumière latérale 28 (fig. 9).

Le sommet de chacune des dents 29 à 31 détermine ainsi la ligne d'obturation d'autant de valves placées « en série » sur le cours dudit flux, et qui ne retiendront que la valeur quasi-maximum de la pression radiale à mesurer, pour la transmettre au manomètre 12 (fig. 1).

S'agissant de connaître la répartition de cette pression radiale non isotrope, on peut, à partir d'un capteur selon figures 8 et 9, comprenant 2 n dents, obtenir n valves indépendantes qui transmettront leur information à autant de manomètres.

A cet effet, en se référant aux figures 4 et 9, un microtube tel que 14 débouchera par une ouverture latérale telle que 16, dans chaque entre-dent pair, tandis qu'une lumière latérale telle que 28 reliera chaque entre-dent impair au collecteur 27.

Ledit flux relatif à un microtube donné se divisera de la sorte pour suivre le profil de chacune des deux dents adjacentes, et rejoindre le collecteur par les deux lumières latérales des entre-dents adjacents.

En variante, les pièces selon figures 6 et 7 peuvent être décalées lors du montage, de manière à obstruer le passage, une dent sur deux, ledit flux ne suivant alors que le profil de la dent non obstruée, ce qui conduit à une mesure mieux localisée.

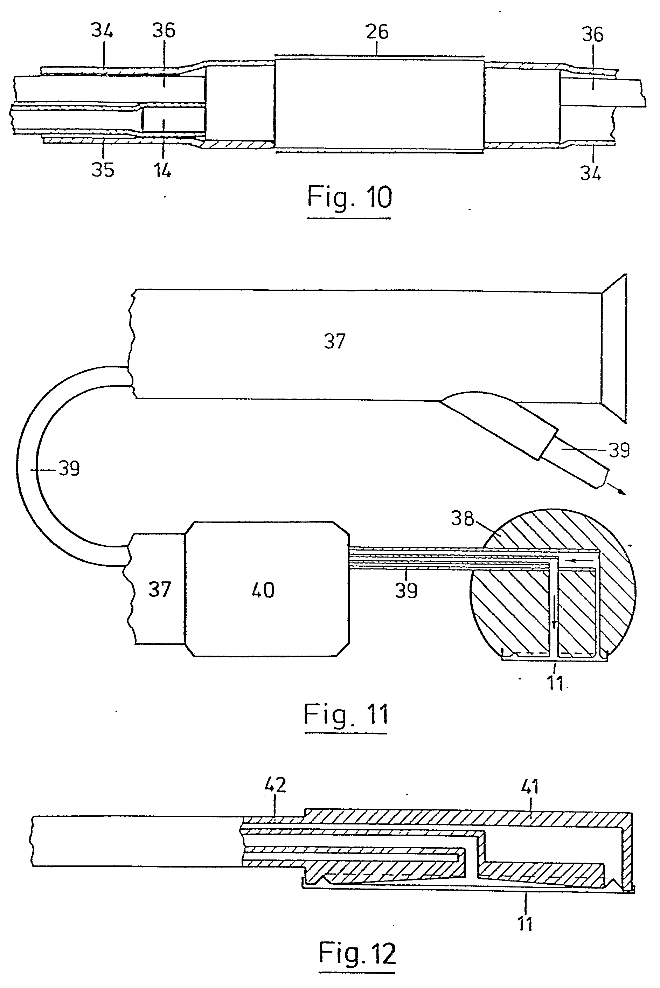

La figure 10 montre le mode d'assemblage des tubes plastiques 34 et 35 sur un capteur à membrane cylindrique 26, appliqué notamment en mesure de pression dans les voies urinaires plus précisément pour mesurer la coordination des pressions entre l'urètre et la vessie et pour déterminer la motilité œsophagienne.

Dans la première de ces applications, la sonde comporte deux capteurs et un cathéter d'injection 36 en transit dans le collecteur 34, tandis que dans la seconde la sonde comporte trois capteurs indépendants et également répartis à raison d'un intervalle d'environ 5 cm entre eux, sous un diamètre total qui ne doit pas dépasser 3,3 mm.

La figure 11 est relative à l'application de la sonde selon l'invention à la mesure de la pression des varices œsophagiennes, à travers un endoscope.

La sonde 39, longue de 2,5 m pour un diamètre de 2 mm, comporte un capteur 38, de forme appropriée, fixé à l'endoscope 37 par une tige rapide et un mécanisme de verrouillage 40... de telle manière que le profil de la membrane latérale plane 11 soit visible à travers l'optique de l'endoscope, pour la positionner contre les varices.

La figure 12 concerne la mesure de la pression intracrânienne des bébés prématurés, par fontanelle interposée.

La sonde 42 se termine par un capteur 41 en forme de galette ou cylindre très court, à membrane latérale plane 11, qui s'appuie sur la fontanelle du prématuré, un simple bonnet de bébé fixant le tout.

Il est bien entendu qu'un même propulseur, tel que 1 (fig. 1), est en mesure d'alimenter plusieurs sondes de ce type, pour la surveillance simultanée continue d'autant de prématurés.

Un autre type de capteur selon la présente invention, a été développé pour mesurer, dans cet ordre d'idée, la pression intracrânienne des adultes, par dure-mère interposée.

Sa structure est celle représentée à la figure 1, mais en y ajoutant un dispositif de fixation dans une épaisseur de boîte crânienne, de préférence par l'intermédiaire d'une bague pré-fixée qui permet l'interchangeabilité de ce capteur pneumatique, et d'un capteur magnétique (brevet CH 579 899).

Ces deux types de capteurs se complètent, en effet, le premier étant utilisé durant les deux jours suivant une opération, tandis que le second est alors implanté, si nécessaire, pour longtemps.

Les capteurs selon l'invention peuvent naturellement être envisagés pour bien d'autres applications, médicales ou non, telles que les examens cardiologiques, par exemple.

Précisons enfin que toutes ces sondes sont embrochables par des connecteurs pneumatiques ad-hoc, qui permettent leur montage en quelques secondes.

Le flux d'air propulsé peut en outre être obtenu à partir d'un réseau d'air comprimé et au moyen d'un mano-détendeur, ladite pression négative étant obtenue à partir du même réseau au moyen d'un Venturi.

## Revendications

1. Dispositif de mesure de pressions physiologiques en des endroits peu accessibles, comprenant une sonde, notamment de mano-cathétérisme, pour transmettre la pression à mesurer, cette sonde comprenant au moins un circuit pneumatique (3, 4, 5, 6, 7 ; 14, 16, 17, 22, 23, 24, 25, 15 ; 14, 16, 28, 27) à flux d'air ou **autre gaz**, ledit circuit comprenant, un tube filiforme (3-4 ; 14) d'entrée, un capteur (10 ; 38 ; 41) de transposition de ladite pression à mesurer, et un collecteur d'évacuation (6, 7 ; 15 ; 27 ; 42 ; 34), ledit capteur étant pourvu d'au moins une valve comprenant, une membrane élastique (11 ; 26), un pavillon (4 ; 18, 19), et un drain (6 ; 22, 23), ladite valve déterminant une ligne d'obturation (5) sur le sommet dudit pavillon, ladite ligne d'obturation engendrant un gradient de pression dudit flux, tel que la pression dans ledit pavillon soit maintenue adaptée à ladite pression à mesurer, le dispositif comprenant en outre un propulseur (1) assurant la circulation dudit flux, un organe (8) d'ajustage du débit dudit flux, et un manomètre (12), caractérisé par le fait que, tout en alimentant ledit capteur par ledit flux, ledit tube filiforme joue également le rôle de transmetteur de ladite pression à mesurer jusqu'en amont (3) de ladite sonde, où elle est mesurée par ledit manomètre, de manière à éviter une tubulure spécifique de mesure et réduire d'autant le diamètre de ladite sonde.

2. Dispositif selon la revendication 1, caractérisé par le fait que ledit collecteur d'évacuation (6, 7 ; 15 ; 27 ; 42 ; 34) est agencé de manière à pouvoir transmettre une pression négative relativement à la pression atmosphérique.

3. Dispositif selon la revendication 1, caractérisé par le fait que ledit tube filiforme (3, 4 ; 14) est disposé à l'intérieur dudit collecteur d'évacuation (6, 7 ; 15 ; 27).

4. Dispositif selon la revendication 1, caractérisé par le fait que ladite sonde est équipée d'un connecteur pneumatique, embrochable.

5. Dispositif selon la revendication 1, caractérisé par le fait que la surface active de ladite membrane (11) est plane.

6. Dispositif selon la revendication 1, caractérisé par le fait que la surface active de ladite membrane (26) est cylindrique.

7. Dispositif selon la revendication 6, caractérisé par le fait que ledit tube filiforme est un microtube (14) fixé à l'intérieur d'un corps tubulaire (15 ; 27) ledit flux passant dans ladite valve par ce microtube et par une ouverture latérale (16) pour pénétrer entre le manteau extérieur dudit corps tubulaire (15) et ladite membrane (26).

8. Dispositif selon la revendication 7, adapté à la mesure de la composante radiale de ladite pression à mesurer, exercée sur une étroite bande annulaire de ladite sonde, caractérisé par le fait que le flux passe de ladite ouverture latérale (16) dans une gorge de distribution (17) aménagée entre deux bagues (18 et 19) qui constituent ledit pavillon, ledit drain étant formé de deux gorges (22 et 23) situées de part et d'autre de ladite gorge de distribution reliées audit collecteur d'évacuation par des lumières latérales (24 et 25).

9. Dispositif selon la revendication 7, caractérisé par le fait que ladite sonde comporte au moins un autre capteur comprenant une valve et un tube filiforme fixé à l'intérieur du corps tubulaire, ledit collecteur d'évacuation étant commun aux capteurs.

10. Dispositif selon les revendications 1 à 6 agencé pour mesurer la pression des varices œsophagiennes à travers un endoscope, caractérisé par le fait que ladite sonde (39) peut s'introduire dans une voie libre dudit endoscope (37), ledit capteur (38) étant fixé audit endoscope par un mécanisme de verrouillage (40).

11. Dispositif selon la revendication 5 agencé pour mesurer la pression intracrânienne des bébés prématurés par fontanelle interposée, caractérisé par le fait que ledit capteur est en forme d'une galette (41), ladite membrane (11) étant disposée sur l'une de ses faces, ledit collecteur d'évacuation (42) sortant, par exemple, latéralement au capteur, de manière à faciliter la fixation dudit capteur sur la fontanelle, par un simple bonnet de bébé.

12. Dispositif selon la revendication 5, agencé pour mesurer la pression intracrânienne par interposition de la dure-mère, caractérisé par le fait que ledit capteur (10) est de forme cylindrique, ladite membrane (11) étant fixée à l'extrémité dudit capteur, selon un plan transversal, ledit capteur étant agencé de manière à permettre sa fixation dans l'épaisseur de la boîte crânienne du patient, ladite membrane (11) affleurant ladite dure-mère.

13. Dispositif selon la revendication 9, agencé pour mesurer la coordination des pressions entre l'urètre et la vessie, caractérisé par le fait que lesdits capteurs sont en nombre de deux, un cathéter d'injection pouvant être logé dans ledit collecteur.

14. Dispositif selon la revendication 9, agencé pour mesurer la motilité œsophagienne, caractérisé par le fait que lesdits capteurs sont en nombre de trois, indépendants et également répartis à raison d'un intervalle d'environ cinq centimètres entre eux.

15. Dispositif selon la revendication 2, caractérisé par le fait que ledit manomètre (12) rend compte de pression à mesurer, telle qu'elle est transmise à ladite sonde, en amont (3) de celle-ci, des moyens de correction étant prévus pour la correction des distorsions de transfert.

16. Dispositif selon la revendication 15, caractérisé par le fait que ledit propulseur consiste en un mini-compresseur spécifique (1).

17. Dispositif selon la revendication 15, caractérisé par le fait que ledit propulseur consiste en un réseau de distribution d'air comprimé, et un mano-détendeur, ladite pression négative d'aspiration étant obtenue à partir dudit réseau et d'un Venturi.

18. Dispositif selon la revendication 15, caractérisé par le fait que lesdits moyens de correction des distorsions de transfert comprennent, des moyens de mise à zéro dudit manomètre, et/ou des moyens de diminution de sa sensibilité, et/ou des moyens d'adaptation de la pression de sortie (2) dudit propulseur (1).

**Claims**

1. Device for measuring physiological pressures in places difficult of access, which comprises a probe, notably a pressure catheter probe, for transmitting the pressure to be measured, said probe comprising at least one pneumatic circuit (3, 4, 5, 6, 7 ; 14, 15, 16, 17, 22, 23, 24, 25, 15 ; 14, 16, 28, 27) of the air-flow or other gas-flow type, said circuit comprising a filiform input tube (3-4 ; 14), a sensor (10 ; 38 ; 41) for transposing said pressure to be measured, and a discharge manifold (6, 7 ; 15 ; 27 ; 42 ; 34), said sensor being provided with at least one valve comprising an elastic diaphragm (11 ; 26), a pavilion (4 ; 18 ; 19) and a drain (6 ; 22, 23), said valve determining a sealing line (5) on top of said pavilion, said sealing line generating a pressure gradient in said flow, such that the pressure in said pavilion be maintained at a value consistent with said pressure to be measured, the device further comprising a propeller (1) for circulating said flow, a member (8) for adjusting the output of said flow, and a pressure gauge (12), characterized by the fact that while supplying said sensor with said flow, said filiform tube is also effective to transmit said pressure to be measured upstream (3) of said probe where it is measured by said pressure gage, in order to dispense with a specific measuring pipe and reduce accordingly the diameter of said probe.

2. The device of claim 1, characterized by the fact that said discharge manifold (6, 7 ; 15 ; 24 ; 72 ; 34) is so arranged that it can transmit a negative pressure in relation to the atmospheric pressure.

3. The device of claim 1, characterized by the fact that said filiform tube (3, 4 ; 14) is disposed inside said discharge manifold (6, 7 ; 15 ; 27).

4. The device of claim 1, characterized by the fact that said probe is provide with a plug-in pneumatic connector.

5. The device of claim 1, characterized by the fact that the operative surface of said diaphragm (11) is flat.

6. The device of claim 1, characterized by the fact that the operative surface of said diaphragm (26) is cylindrical.

7. The device of claim 6, characterized by the fact that said filiform tube is a microtube (14) secured within a tubular body (15 ; 27), said flow passing into said valve through said microtube and through a lateral opening (16) before penetrating between the outer mantel of said tubular body (15) and said diaphragm (26).

8. The device of claim 7, adapted to measure the radial component of said pressure to be measured, exerted against a narrow annular band of said probe, characterized by the fact that the flow emerging from said lateral opening (16) penetrates into a distribution groove (17) formed between two rings (18, 19) constituting said pavilion, said drain consisting of two grooves (22, 23) disposed on either side of said distribution groove and connected to said discharge manifold via side ports (24, 25).

9. The device of claim 7, characterized by the fact that said probe comprises at least another sensor comprising a valve and a filiform tube secured within the tubular body, said discharge manifold being common to said sensors.

10. The device of claim 1 to 6, adapted to measure the pressure of oesophagian varices through an endoscope, characterized by the fact that said probe (39) can be introduced into a free passage of said endoscope (37), said sensor being secured to said endoscope by means of a locking mechanism (40).

11. The device of claim 5, adapted to measure the intracranial pressure of babies through the medium of the fontanelle, characterized by the fact that said sensor has a cake-liked configuration (41), said diaphragm (11) being disposed on one of its faces, said discharge manifold (42) emerging for example laterally from the sensor in order to facilitate the fixing of said sensor to the fontanelle by using a simple baby cap.

12. The device of claim 5, adapted to measure the intracranian pressure with the interposition of the dura mater, characterized by the fact that said sensor (10) has a cylindrical configuration, said diaphragm being secured to the end of said sensor in a transverse plane, said sensor being so arranged as to permit the fixing thereof in the thickness of the patient's brain-pan, said diaphragm being flush with said dura mater.

13. The device of claim 9, adapted to measure the coordination of pressures between the urethra and the bladder, characterized by the fact that said sensors are two in number, and that an injection catheter can be housed in said manifold.

14. The device of claim 9, adapted to measure the oesophagian motility, characterized by the fact that said sensors are three in number, independent and equally distributed by reason of a gap of about five centimeters between them.

15. The device of claim 2, characterized by the fact that said pressure gage (12) displays the pressure to be measured, such as transmitted to said sensor, upstream thereof (3), correction means being provided for correcting the transfer distortions.

16. The device of claim 15, characterized by the fact that said propeller consists of a specific mini-compressor (1).

17. The device of claim 15, characterized by the fact that said propeller consists of a compressed-air supply line and a pressure-reducing gage, said negative suction pressure being obtained from said line via a Venturi.

18. The device of claim 15, characterized by the fact that said means for correcting transfer distortions comprise means for resetting said pressure gage, and/or means for reducing its sensitivity, and/or means for adapting the output pressure (2) of said propeller (1).

**Ansprüche**

1. Vorrichtung zur Messung von physiologischen Drücken an schwer zugänglichen Stellen mit einer Sonde, insbesondere für Druckkatheterisierung, zur Übertragung des zu messenden Druckes mittels wenigstens eines pneumatischen Kreilsaufes (3, 4, 5, 6, 7 ; 14, 16, 17, 22, 23, 24, 25, 15 ; 14, 16, 28, 27) aus einem Luft- oder einem anderen Gasstrom durch eine fadenförmige Zuführungsröhre (3, 4 ; 14) und einen Fühler (10 ; 38 ; 41) zur Übertragung des zu messenden Druckes sowie durch eine Sammelableitung (6, 7 ; 15 ; 27 ; 42 ; 34), wobei der Fühler wenigstens ein Ventil aus einer elastischen Membran (11 ; 26), einen Trichter (4 ; 18, 19) und eine Ableitung (6 ; 22, 23) aufweist und dieses Ventil am oberen Trichterrand eine Verschlusslinie (5) bildet, welche in dieser Strömung einen solchen Druckgradienten erzeugt, dass der Druck in diesem Trichter an den zu messenden Druck angepasst gehalten wird, ferner mit einer Strömungsquelle (1) zur Erzeugung einer Strömung, einer Einrichtung (8) zur Einstellung der Strömungsmenge und einem Manometer (12), dadurch gekennzeichnet, dass beim Strömen dieses Gasstromes zu dem Fühler die fadenförmige Röhre gleichfalls als Überträger des zu messenden Druckes stromaufwärts bis zum Eintritt (3) in die Sonde wirkt, wo er durch das Manometer gemessen wird, um eine besondere Messleitung zu vermeiden und den Durchmesser dieser Sonde entsprechend herabzusetzen.

2. Vorrichtung nach Anspruch 1, dadurch gekennzeichnet, dass die Sammelableitung (6, 7 ; 15 ; 27 ; 42 ; 34) so eingerichtet ist, dass sie einen negativen Druck bezüglich des Atmosphärendruckes übertragen kann.

3. Vorrichtung nach Anspruch 1, dadurch gekennzeichnet, dass die fedenförmige Röhre (3, 4 ; 14) innerhalb der Sammelableitung (6, 7 ; 15 ; 27) angeordnet ist.

4. Vorrichtung nach Anspruch 1, dadurch gekennzeichnet, dass die Sonde mit einem anschliessbaren pneumatischen Anschluss ausgestattet ist.

5. Vorrichtung nach Anspruch 1, dadurch gekennzeichnet, dass die Messfläche der Membran (11) eben ist.

6. Vorrichtung nach Anspruch 1, dadurch gekennzeichnet, dass die Messfläche der Membran (26) zylindrisch ist.

7. Vorrichtung nach Anspruch 6, dadurch gekennzeichnet, dass die fadenförmige Röhre als Mikroröhre (14) ausgebildet und im Innern eines Rohres (15 ; 27) befestigt ist, wobei die Strömung in dem Ventil durch diese Mikroröhre und eine seitliche Öffnung (16) geht und zwischen dem Aussenmantel des Rohres (15) und der Membran (26) eintritt.

8. Vorrichtung nach Anspruch 7 zur Messung der Radialkomponente des zu messenden Druckes auf einen schmalen Ringstreifen der Sonde, dadurch gekennzeichnet, dass die Strömung von der seitlichen Öffnung (16) in eine Verteilernut (17) zwischen zwei Ringen (18, 19) geht, welche den Trichter bilden, wobei die Ableitung von zwei Nuten (22, 23) beiderseits der Verteilernut gebildet wird, die mit der Sammelableitung über seitliche Öffnungen (24, 25) verbunden sind.

9. Vorrichtung nach Anspruch 7, dadurch gekennzeichnet, dass die Sonde wenigstens einen weiteren Fühler mit einem Ventil und einer fadenförmigen Röhre im Inneren des Rohres aufweist und die Sammelableitung an die Fühler angeschlossen ist.

10. Vorrichtung nach Anspruch 1 bis 6 zur Messung des Druckes von Speiseröhrenkrampfadern durch ein Endoskop, dadurch gekennzeichnet, dass die Sonde (39) in einen freien Kanal des Endoskopes (37) einführbar und der Fühler (38) an dem Endoskop mittels einer Verriegelungseinrichtung (40) befestigt ist.

11. Vorrichtung nach Anspruch 5 zur Messung des Innenschädeldruckes von frühgeborenen Kleinkindern durch die dazwischenliegende Fontanelle, dadurch gekennzeichnet, dass der Fühler die Form eines flachen Gehäuses (41) mit der Membran (11) auf einer seiner Seiten aufweist und die Sammelableitung beispielsweise seitlich am Fühler herausführt, um die Befestigung des Fühlers auf der Fontanelle mittels einer einfachen Kleinkinderkappe zu erleichtern.

12. Vorrichtung nach Anspruch 5 zur Messung des Innenschädeldruckes durch die dazwischenliegende Gehirnhaut, dadurch gekennzeichnet, dass der Fühler (10) von zylindrischer Form ist und die Membran (11) in einer Querebene am Ende des Fühlers sitzt, wobei der Fühler in einer Weise ausgebildet ist, die seine Befestigung in der Hirnschale des Patienten mit der Membran (11) an der Gehirnhaut ermöglicht.

13. Vorrichtung nach Anspruch 9 zur Messung der Druckkoordination zwischen der Harnröhre und der Blase, dadurch gekennzeichnet, dass die Fühler in zweifacher Anzahl vorhanden sind und ein Injektionskatheter in der Sammelleitung anbringbar ist.

14. Vorrichtung nach Anspruch 9 zur Messung der Speiseröhrenbeweglichkeit, dadurch gekennzeichnet, dass die Fühler in dreifacher Anzahl vorhanden und unabhängig gleichmässig mit einem Abstand von etwa 5 cm untereinander verteilt sind.

15. Vorrichtung nach Anspruch 2, dadurch gekennzeichnet, dass das Manometer (12) den zu messenden Druck so angibt, wie er an die Sonde stromaufwärts an deren Eintritt (3) übertragen wird, wobei Korrektureinrichtungen zur Korrektur von Übertragungsverzerrungen vorgesehen sind.

16. Vorrichtung nach Anspruch 15, dadurch gekennzeichnet, dass die Strömungsquelle aus einem besonderen Minikompressor (1) besteht.

17. Vorrichtung nach Anspruch 15, dadurch gekennzeichnet, dass die Strömungsquelle aus einem Druckluftverteilernetz und einem Druckminderer besteht und der negative Ansaugdruck von diesem Netz und einem Venturi-Rohr erhalten wird.

18. Vorrichtung nach Anspruch 15, dadurch gekennzeichnet, dass die Korrektureinrichtungen für die Übertragungsverzerrungen aus Einrichtungen zur Nullrückstellung des Manometers und/oder zur Verminderung seiner Empfindlichkeit und/oder zur Anpassung des Ausgangsdruckes (2) der Strömungsquelle (1) bestehen.

Fig. 1

Fig. 2

Fig. 3

Fig. 4

Fig. 5

Fig. 6

Fig. 7

Fig. 8

Fig. 9

34    36                    26                    36

35    14

Fig. 10

37

39

39

38

37    40    39    11

Fig. 11

42                    41

11

Fig. 12